# EUROPEAN PATENT SPECIFICATION

(11) **EP 1 989 003 B1**
(45) Date of publication and mention of the grant of the patent: **11.01.2012**
(21) Application number: 07701871.1
(22) Date of filing: 13.02.2007
(51) Int. Cl.: B05C 17/005, B05C 17/01

(54) **DISPENSING APPLIANCE FOR A DOUBLE SYRINGE**
AUSGABEVORRICHTUNG FÜR EINE DOPPELSPRITZE
DISPOSITIF DE DISTRIBUTION POUR SERINGUE DOUBLE

(30) Priority: 24.02.2006 CH 288062006
(43) Date of publication of application: 12.11.2008
(73) Proprietor: Sulzer Mixpac AG, 9469 Haag (CH)
(72) Inventor: KELLER, Wilhelm, A., CH-6402 Merlischachen (CH)
(74) Representative: Sulzer Management AG
(86) International application number: PCT/CH2007/000071
(87) International publication number: WO 2007/095769

(56) References cited:
- EP-A- 0 791 403
- EP-A1- 0 037 393
- WO-A-94/07420
- DE-A1- 1 938 028
- US-A- 4 874 368
- US-A- 5 005 735

## Description

Present invention relates to a dispensing appliance according to the preamble of claim 1.

Generally, double syringes or double cartridges with a static mixer are used for mixing and dispensing two-component materials. A double syringe system generally consists of two parallelly arranged storage cylinders, two pistons, a common closure, and a double plunger. To dispense the content, the pistons are pushed forward by means of the double plunger.

EP-A1-0 037 393 describes an apparatus including a plurality of standardized one-way syringe bodies accommodating plungers, and holding means provided for the plurality of syringe bodies, and a guide rod penetrating the holding means, and a common actuating means being guided along said guide rod provided for the plungers of the syringe bodies.

Particularly in smaller double syringes for single use, the conventional dispensing by means of the dual plungers may result in handling problems because of the small dimensions, and it is therefore the object of the present invention to provide a dispensing appliance that allows a comfortable dispensing also of small double syringes. This object is attained by the dispensing appliance according to claim 1.

The invention will be explained in more detail hereinafter with reference to drawings of exemplary embodiments.
- Fig. 1: shows a perspective view of a double syringe with a mixer and of a dispensing appliance not forming part of the present invention,
- Fig. 2: shows the double syringe of Fig. 1 inserted in the dispensing appliance of Fig. 1,
- Fig. 3: shows the dispensing appliance of Fig. 2,
- Fig. 4: shows a partly sectioned view according to arrow IV in Fig. 3,
- Fig. 5: shows a section according to line V - V in Fig. 4,
- Fig. 6: shows an enlarged detail of the plunger end according to Fig. 4,
- Fig. 7: shows a double syringe in the embodiment of the dispensing appliance according to the invention, and
- Fig. 8: shows the dispensing appliance of Fig. 7 in a perspective view and individually.

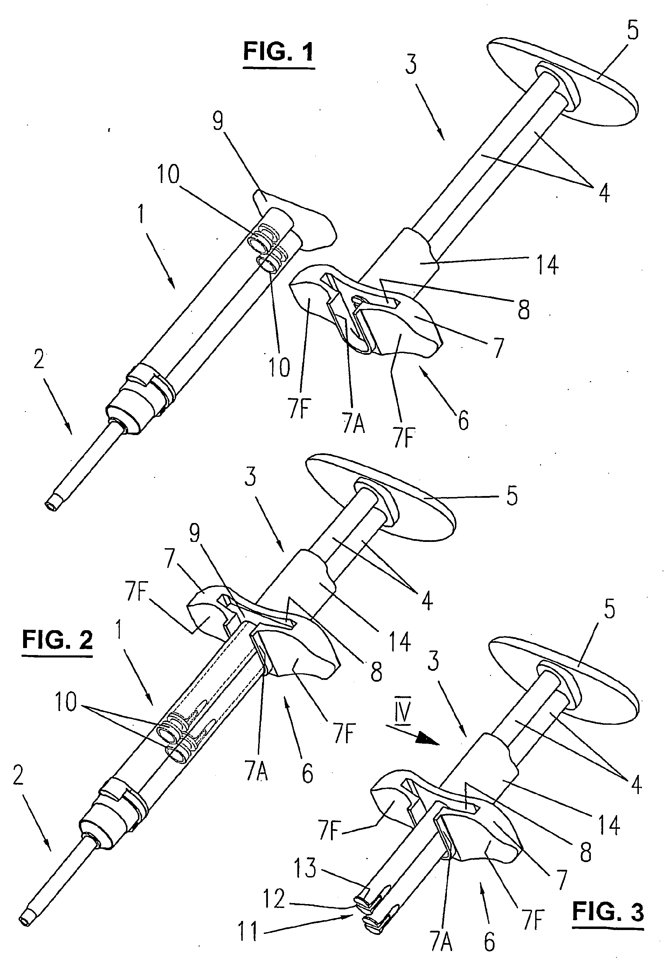

Fig. 1 shows double syringe 1 with a mixer 2 as well as a dispensing appliance 3 not forming part of the invention with double plunger 4, connected by thrust plate 5, as well as a flange holder 6 having a finger rest grip 7 with finger rests 7F and a guiding slot 8 for receiving retaining flange 9 of double syringe 1. On the syringe side of the flange holder, a collar 7A is provided that serves for guiding the syringe and comprises a syringe snap-in retainer for the container that is inserted first, which is in the case of container volumes that are different from 1:1 the larger container. Furthermore, in Fig. 1, pistons 10 in the syringe are shown.

Fig. 1 shows how the filled and operational syringe 1 can be introduced with its retaining flange 9 into guiding slot 8 of the flange holder of the dispensing appliance. The internal contour of guiding slot 8 is adapted to the coded external contour of retaining flange 9, thereby allowing to prevent that non-system syringes may be used and to ensure, in syringes having containers whose volumetric ratio respectively diameter ratio is different from 1:1, that the syringe is inserted into the guiding slot in the correct position.

In Fig. 2, the syringe is in place on the dispensing appliance and a part of the content of the double syringe has already been dispensed, as follows from the position of the pistons and plungers.

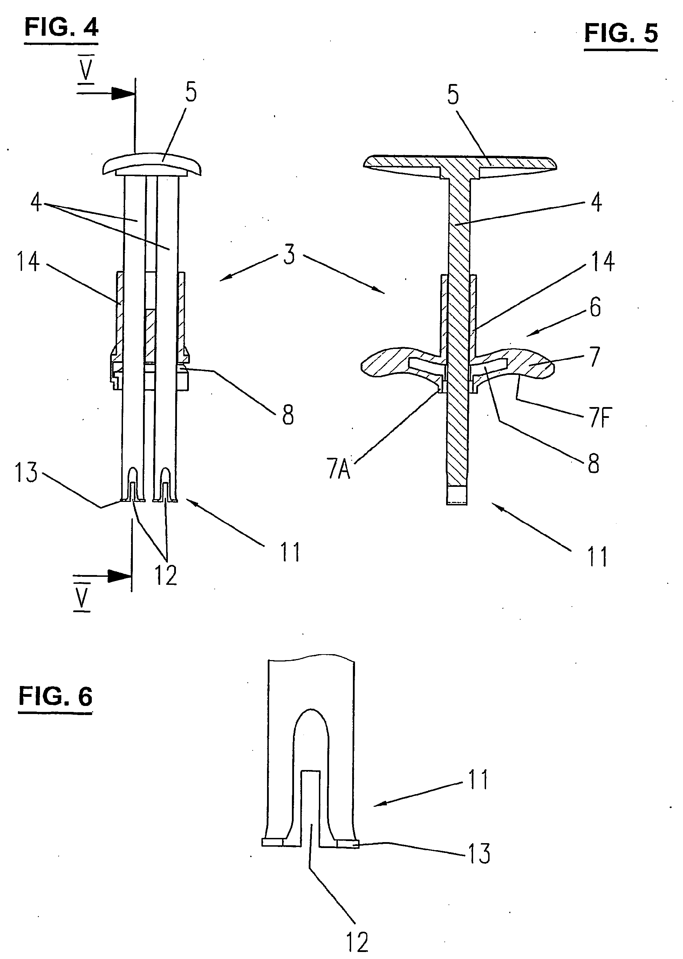

In Fig. 3 it is apparent that the forward ends 11 of double plungers 4 have an incision 12 to provide a certain elasticity of these ends. In the enlarged illustration of Fig. 6 of a plunger end it is visible that the end spreads out slightly resiliently and has a circumferential bulge 13 such that the plunger ends have a slightly larger diameter than the remainder of the plungers. This enlargement of the circumference by the circumferential bulge 13 and the slight spreading at ends 11 of double plungers 4 have the effect that when the double plunger is pulled back, it is first held back and can only be withdrawn from plunger guide 14 by applying an increased force. When a normal force is applied, the circumferential bulge provides a sufficient safety against a complete withdrawal of the plunger from the flange holder.

In Fig. 4 it is shown that the double plungers are guided by a sufficiently long plunger guide 14 that is arranged on the finger rest grip and extends toward the thrust plate. This allows a precise application, which is important especially in smaller double syringes.

Fig. 5 illustrates flange holder 6 with finger rest grip 7 and its finger rests 7A as well as guiding slot 8, the latter guiding slot being shaped for receiving the coded and curved retaining flange of the double syringe.

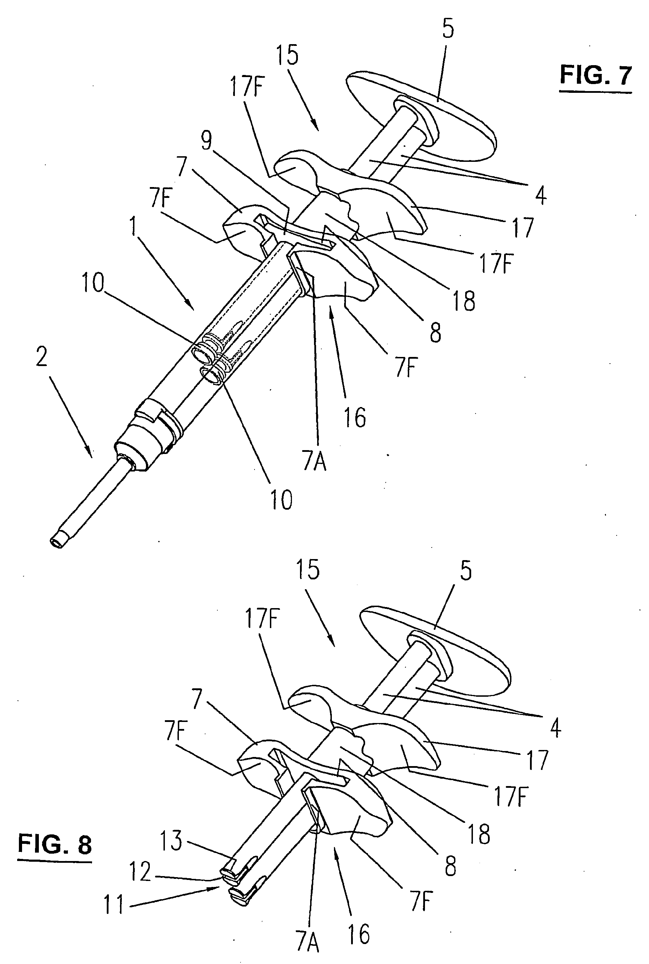

Figs. 7 and 8 illustrate an embodiment according to the invention where flange holder 16 of dispensing appliance 15 has a second finger rest grip 17 with finger rests 17F that is arranged on plunger guide 18 at some distance from the first finger rest grip 7. By this second finger rest grip, the distance to the thrust plate is reduced, thereby allowing a comfortable operation of the syringe also by people having smaller hands, especially in the case of longer syringes. The remaining parts of the dispensing appliance are the same as in the preceding exemplary embodiment.

The term double syringe is also meant to include a double cartridge. Moreover, instead of a mixer, any other accessory such as a spray nozzle or another accessory part may be connected to the double syringe.

## Claims

1. Dispensing appliance for a double syringe or cartridge, including double plungers (4) connected by a thrust plate (5) as well as a flange holder ( 16) having a guiding slot (8) for receiving the retaining flange (9) of the double syringe (1) or double cartridge and at least one finger rest grip (7), wherein the flange holder (6, 16) has a plunger guide (14, 18) on its side facing away from the double syringe or double cartridge,
**characterised in that** the flange holder (16) has a second finger rest grip (17) that is arranged on the plunger guide (18) at a distance from the first finger rest grip (7) on the thrust plate side thereof.

2. Dispensing appliance according to claim 1, **characterised in that** the ends (11) of the plungers (4), which open out elastically, have an incision (12) and a circumferential bulge (13).

3. Dispensing appliance according to claim 1 or 2, **characterised in that** the guiding slot (8) in the flange holder (6, 16) has a coded internal contour for receiving the coded contour of the retaining flange (9) of the double syringe or double cartridge in an unequivocal position.

## Patentansprüche

1. Austraggerät für eine Doppelspritze oder -kartusche, enthaltend Doppelstössel (4), die über eine Druckplatte (5) verbunden sind und einen Flanschhalter (16), der einen Führungsschlitz (8) hat, um einen Rückhalte-Flansch (9) der Doppelspritze (1) oder Doppelkartusche aufzunehmen und zumindest einen Fingerauflage-Griff (7), wobei der Flanschhalter (6, 16) eine Stösselführung (14, 18) auf der Seite, die von der Doppelspritze oder Doppelkartusche weggerichtet ist, aufweist, **dadurch gekennzeichnet, dass** der Flanschhalter (16) einen zweiten Fingerauflagegriff (17) aufweist, der auf der Stösselführung (18) in einem Abstand vom ersten Fingerauflage-Griff (7) auf der Seite der Druckplatte angebracht ist.

2. Austraggerät nach Anspruch 1, **dadurch gekennzeichnet, dass** die Enden (11) der Stössel (4), die federnd nach aussen öffnen, einen Einschnitt (12) und einen umlaufenden Wulst (13) aufweisen.

3. Austraggerät nach Anspruch 1 oder 2, **dadurch gekennzeichnet, dass** der Führungsschlitz (8) im Flanschhalter (6, 16) eine kodierte Innenkontur aufweist, um die kodierte Kontur des Rückhalteflansches (9) der Doppelspritze oder Doppelkartusche in einer eindeutigen Lage aufzunehmen.

## Revendications

1. Dispositif de distribution pour une seringue ou cartouche double, comprenant des plongeurs doubles (4) reliés par une plaque de poussée (5) ainsi qu'un organe de retenue de bride (16) comportant une fente de guidage (8) pour recevoir la bride de retenue (9) de la seringue double (1) ou de la cartouche double et au moins un support d'appui des doigts (7), où l'organe de retenue de bride (6, 16) possède un guide de plongeur (14, 18) sur son côté éloigné de la seringue double ou cartouche double, **caractérisé en ce que** l'organe de retenue de bride (16) possède un deuxième support d'appui des doigts (17) qui est agencé sur le guide de plongeur (18) à une distance du premier support d'appui des doigts (7) sur le côté de la plaque de poussée de celui-ci.

2. Dispositif de distribution selon la revendication 1, **caractérisé en ce que** les extrémités (11) des plongeurs (4), qui s'ouvrent élastiquement, ont une incision (12) et un renflement circonférentiel (13).

3. Dispositif de distribution selon la revendication 1 ou 2, **caractérisé en ce que** la fente de guidage (8) dans l'organe de retenue de bride (6, 16) possède un contour intérieur codé pour recevoir le contour codé de la bride de retenue (9) de la seringue double ou de la cartouche double dans une position univoque.
